(19) Europäisches Patentamt
European Patent Office
Office européen des brevets

(11) EP 0 928 785 B1

(12) EUROPÄISCHE PATENTSCHRIFT

(45) Veröffentlichungstag und Bekanntmachung des Hinweises auf die Patenterteilung:
**19.06.2002 Patentblatt 2002/25**

(51) Int Cl.[7]: **C07C 263/10**

(21) Anmeldenummer: **98124802.4**

(22) Anmeldetag: **29.12.1998**

(54) **Verfahren zur Phosgenierung von Aminen in der Gasphase unter Einsatz von Mikrostrukturmischern**

Process for the phosgenation of amines in the gas phase using microstructure mixers

Procédé pour la phosgénation d'amines en phase gazeuse utilisant des mélangeurs à microstructure

(84) Benannte Vertragsstaaten:
**BE DE ES FR GB IT NL**

(30) Priorität: **09.01.1998 DE 19800529**

(43) Veröffentlichungstag der Anmeldung:
**14.07.1999 Patentblatt 1999/28**

(73) Patentinhaber:
- **Bayer Aktiengesellschaft**
  **51368 Leverkusen (DE)**
- **Forschungszentrum Karlsruhe GmbH**
  **76133 Karlsruhe (DE)**

(72) Erfinder:
- **Becker, Gernot Dr.**
  **41540 Dormagen (DE)**
- **Fischer, Konrad Dr.**
  **51519 Odenthal (DE)**
- **Flink, Andreas**
  **41539 Dormagen (DE)**
- **Herrmann, Erhard Dr.**
  **Fracc. Club de Golf Chiluca (MX)**
- **Weismantel, Lothar Dr.**
  **51065 Köln (DE)**
- **Wiessmeier, Georg Dr.**
  **51061 Köln (DE)**
- **Schubert, Klaus Dr.**
  **76227 Karlsruhe (DE)**
- **Fichtner, Maximilian Dr.**
  **68723 Oftersheim (DE)**

(74) Vertreter: **Steiling, Lothar, Dr.**
**Bayer AG**
**Konzernbereich RP**
**Patente und Lizenzen**
**51368 Leverkusen (DE)**

(56) Entgegenhaltungen:
**EP-A- 0 322 647** **WO-A-95/30476**
**DE-A- 2 950 216** **DE-A- 19 541 266**

- **DATABASE WPI Section Ch, Week 8217 Derwent Publications Ltd., London, GB; Class E19, AN 82-34302E XP002100326 & JP 57 048954 A (MITSUI TOATSU CHEM INC) , 20. März 1982**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 928 785 B1

## Beschreibung

Die vorliegende Erfindung betrifft ein Verfahren zur Herstellung von Isocyanaten durch Phosgenierung der entsprechenden Amine in der Gasphase unter Einsatz von Mikrostruktur-Mischern zur schnellen Vermischung der Edukte.

Zur Durchführung einer chemischen Reaktion in kontinuierlicher Fahrweise müssen die Reaktionspartner kontinuierlich einem chemischen Reaktor zugeführt werden und mit Hilfe eines Mischorgans (Mischers) innig in Kontakt gebracht, d.h. gut vermischt werden. Im Regelfall laufen im Reaktor bei Kontakt der Reaktanden mehrere Reaktionen, sogenannte Haupt- und Nebenreaktionen ab. Dabei ist es Ziel des Verfahrensingenieurs, die Reaktionen und damit auch die Vermischung so zu führen, daß selektiv eine möglichst hohe Ausbeute an erwünschtem Produkt erzielt wird.

Die Güte der Vermischung und der Einfluß des Mischorgans auf die Ausbeute an erwünschtem Produkt hängt dabei in großem Maße vom Verhältnis der durch die Reaktionskinetik gegebenen chemischen Reaktionsgeschwindigkeit zur Mischgeschwindigkeit ab. Handelt es sich bei den chemischen Reaktionen um langsame Reaktionen, so ist die chemische Reaktion in der Regel wesentlich langsamer als die Vermischung. Die Brutto-Reaktionsgeschwindigkeit und die Ausbeute an erwünschtem Produkt wird dann durch den langsamsten Schritt, nämlich die Kinetiken der ablaufenden chemischen Reaktionen, und dazu durch das globale Vermischungsverhalten (Verweilzeitverteilung, Makromischung) des verwendeten chemischen Reaktors bestimmt. Liegen die chemischen Reaktionsgeschwindigkeiten und die Vermischungsgeschwindigkeit in der gleichen Größenordnung, so kommt es zu komplexen Wechselwirkungen zwischen den Kinetiken der Reaktionen und dem lokalen, durch die Turbulenz bestimmten Vermischungsverhalten im verwendeten Reaktor und am Mischorgan (Mikromischung). Tritt der Fall ein, daß die chemischen Reaktionsgeschwindigkeiten wesentlich schneller sind als die Mischgeschwindigkeit, so werden die Brutto-Geschwindigkeiten der ablaufenden Reaktionen und die Ausbeuten im wesentlichen durch die Vermischung, d.h. durch das lokale, zeitabhängige Geschwindigkeits- und Konzentrationsfeld der Reaktanden, d.h. die Turbulenzstruktur im Reaktor bzw. am Mischorgan bestimmt (Brodkey, Turbulence in Mixing Operations, Academic Press 1975).

Nach dem Stand der Technik werden zur Durchführung schneller Reaktionen in kontinuierlicher Fahrweise eine Reihe von Mischorganen eingesetzt. Man kann hier grundsätzlich unterscheiden zwischen dynamischen Mischern, wie z.B. Rührern, Turbinen oder Rotor-Stator-Systemen, statischen Mischern, wie z.B. Kenics-Mischern, Schaschlik-Mischern oder SMV-Mischern und Strahlmischern, wie z.B. Düsenmischern oder T-Mischern (Chem. Ing. Tech. MS 1708/88, Fortschr. Verf. Technik 23, 1985, 373, Ind. Eng. Chem. Res. 26, 1987, 1184).

Bevorzugt werden zur schnellen Vermischung der Ausgangsstoffe bei schnellen Reaktionen mit unerwünschten Folge- bzw. Nebenreaktionen Düsenmischer eingesetzt, Dies gilt insbesondere für Reaktionen, die in der Gasphase ablaufen.

Die Herstellung von Isocyanaten durch Umsetzung von Aminen in der Gasphase ist seit langem bekannt. Technische Bedeutung hat sie jedoch erst erlangt, seitdem ein Verfahren bekannt ist, in dem die Probleme der teilweisen Zersetzung polyfunktioneller Amine bei der Verdampfung und der Neigung zur Bildung von Polymeren während der Phosgenierung ausgeräumt sind (EP-A 289 840).

In den EP-A 0 289 840, 0 676 392 und 0 749 958 werden Verfahren zur Herstellung aliphatischer Di- und Triisocyanate aus den entsprechenden Di- bzw. Triaminen durch Phosgenierung in der Gasphase beschrieben. Hier werden die Eduktgasströme zur Reaktion in einen Rohrreaktor geleitet, wobei die Vermischung der Reaktanden am Eintritt in den Rohrraum durch Düsen oder eine Kombination aus Düse und Ringspalt zwischen Düse und Rohr erfolgt. Für die Vermischung wird hier als wesentliches Kriterium eine Reynoldszahl von $Re \geq 4700$ im Rohr angegeben.

In der EP-A 0 570 799 wird zur Vermischung der Edukte bei der Herstellung aromatischer Diisocyanate durch Phosgenierung in der Gasphase ein Strahlmischer eingesetzt. In der EP-A 0 699 657 wird ein Mehrdüsen-Injektionssystem zur Vermischung der Edukte beschrieben.

Bei den beschriebenen Strahl- bzw. Düsenmischern wird eine der beiden Ausgangskomponenten mit hoher Strömungsgeschwindigkeit in die andere Komponente verdüst. Dabei wird die kinetische Energie des eingedüsten Strahles im wesentlichen hinter der Düse dissipiert, d.h. durch turbulenten Zerfall des Strahles in Wirbel und weiteren turbulenten Zerfall der Wirbel in immer kleinere Wirbel in Wärme umgewandelt. In den Wirbeln sind jeweils die Ausgangskomponenten enthalten, die in den Fluidballen nebeneinander vorliegen (Makromischung). Zwar tritt an den Rändern dieser zunächst größeren Strukturen zu Beginn des turbulenten Wirbelzerfalls eine geringe Mischung durch Diffusion auf Die vollständige Vermischung wird jedoch erst erreicht, wenn der Wirbelzerfall soweit fortgeschritten ist, daß mit Erreichen von Wirbelgrößen in der Größenordnung des Konzentrations-Mikromaßes (Batchelor-Länge) (J. Fluid Mech. 5, 1959, 113, Chem. Eng. Sci. 43, 1988, 107) die Diffusion schnell genug ist, um die Ausgangskomponenten in den Wirbeln vollständig miteinander zu vermischen. Die für die vollständige Vermischung nötige Mischzeit hängt neben den Stoffdaten und der Geometrie der Apparatur im wesentlichen von der spezifischen Energiedissipationsrate ab.

Somit vergeht bei nach dem Stand der Technik verwendeten Düsen- oder Strahlmischern bis zur vollständigen Vermischung durch Diffusion immer die Zeit

des Wirbelzerfalls. Für sehr schnelle Reaktionen bedeutet dies, daß entweder sehr hohe Energiedissipationsraten eingestellt werden müssen, um unerwünschte Neben- und Folgereaktionen zu vermeiden oder bei Reaktionen mit noch größeren Reaktionsgeschwindigkeiten die entsprechenden Reaktionen nicht optimal, d.h. nur unter Neben- bzw. Folgeproduktbildung, durchgeführt werden.

Ausgehend von diesem Stand der Technik besteht die Aufgabe der Erfindung in der Bereitstellung eines Verfahrens für die Herstellung von Isocyanaten, vorzugsweise Diisocyanaten, Triisocyanaten und Ether (poly)isocyanaten durch Gasphasenphosgenierung von Aminen, vorzugsweise Diaminen, Triaminen und Ether(poly)aminen, mit dem die Mischung der Edukte schnell erfolgt und die Bildung von Folge- bzw. mit dem die Mischung der Edukte schnell erfolgt und die Bildung von Folge- bzw. Nebenprodukten unterdrückt bzw. reduziert wird. Dabei muß erreicht werden, daß die Edukte schnell homogen miteinander gemischt werden, so daß innerhalb kürzester Zeit keine örtlichen und keine zeitlichen Überkonzentrationen der Edukte mehr auftreten. Gleichzeitig soll eine vollständige Reaktion der Edukte erfolgen. Insbesondere soll die Entstehung von unerwünschten festen Nebenprodukten, die zu Verstopfungen und Ablagerungen führen können, vermieden werden. Ein Gesamtverfahren zur Herstellung (cyclo)aliphatischer Diisocyanate aus (cyclo)aliphatischen Diaminen und Phosgen, in dem die schnelle Vermischung der Reaktanden ein wichtiger Teilschritt ist, wird in der EP-A 0 289 840 beschrieben. Darauf wird hier ausdrücklich Bezug genommen. Ein ganz ähnliches Gesamtverfahren zur Herstellung aromatischer Diisocyanate aus aromatischen Diaminen findet sich in EP 0 570 799.

Die Aufgabe wird erfindungsgemäß durch ein Verfahren gelöst, bei dem die Edukte Amin und Phosgen sowie ein eventuell eingesetztes Inertgas durch eine Mischvorrichtung gemischt werden, deren charakteristische Mischlänge sehr klein ist.

Die Erfindung betrifft daher ein Verfahren zur Herstellung von Isocyanaten, vorzugsweise Diisocyanaten, Triisocyanaten und Ether(poly)isocyanaten durch Phosgenierung der entsprechenden Amine, vorzugsweise Diamine, Triamine und Ether(poly)amine in der Gasphase unter Einsatz eines Mikrostrukturmischers zur schnellen Vermischung des Edukts, Amin, vorzugsweise Diamin oder Triamin oder Ether(poly) amin mit dem Edukt Phosgen, dadurch gekennzeichnet, daß die Mischung mit einem Mikrostrukturmischen erfolgt, bei dem die Edukte Phosgen und Amin durch eine ihnen jeweils zugeordnete Schar von Mikrokanälen in räumlich getrennte Fluidfäden aufgeteilt werden, und die Fluidfäden der Edukte Amin und Phosgen als Freistrahlen mit für das jeweilige Edukt gleichen Strömungsgeschwindigkeiten in den Misch/Reaktionsraum austreten, wobei jeder Freistrahl des Edukts Phosgen in unmittelbarer Nachbarschaft zu einem Freistrahl des Edukts Amin in den Misch- und Reaktionsraum geführt wird und sich die benachbarten Freistrahlen durch Diffusion und/oder Turbulenz vermischen.

Im erfindungsgemäßen Verfahren werden Diisocyanate der Formel (I)

$$OCN\text{-}R\text{-}NCO \qquad (I),$$

durch Phosgenierung der entsprechenden Diamine der Formel (II)

R für einen (cyclo-)aliphatischen Kohlenwasserstoffrest mit bis zu 15, vorzugsweise 3 bis 13 Kohlenstoffatomen steht mit der Maßgabe, daß zwischen den beiden Aminogruppen mindestens zwei Kohlenstoffatome angeordnet sind,

erhalten.

Unter einem (cyclo-)aliphatischen Rest sind hierbei sowohl aliphatische als auch cycloaliphatische als auch aliphatisch-cycloaliphatische Reste zu verstehen, wobei sich aliphatisch bzw. cycloaliphatisch jeweils auf die Natur des mit den Aminogruppen verknüpften Kohlenstoffatoms bezieht. Typische Beispiele geeigneter Diamine sind Hexamethylen-1,6-diamin (HDA), 1-Amino-3,3,5-trimethyl-5-aminomethyl-cyclohexan (IPDA), 4,4'-Diaminodicyclohexylmethan, Tetramethylen-1,4-diamin (BDA), Pentan-1,3-diamin sowie 2,4-Diamino-1-methylcyclohexan und 2,6-Dimino-1-methylcyclohexan (H6-TDA).

In gleicher Weise kann R für einen Kohlenwasserstoffrest mit wenigstens einem aromatischen System stehen. Dieses aromatische System kann z. B. durch Reste wie Alkylgruppen, Halogenatome und Ethergruppen substituiert sein. Typische Beispiele geeigneter Diamine sind Toluylen-2,4-diamin und Toluylen-2,6-diamin (TDA) sowie Diphenylmethan-4,4'-diamin (MDA).

Erfindungsgemäß können weiterhin Triisocyanate der Formel (III)

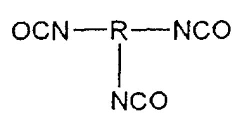

(III),

durch Phosgenierung der entsprechenden Triamine der Formel (IV)

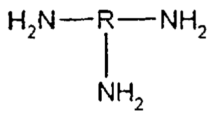

(IV),

in welchen

R für einen gesättigten (cyclo)aliphatischen Kohlenwasserstoffrest mit bis zu 22, vorzugsweise 7 bis 11 Kohlenstoffatomen steht, mit der Maßgabe, daß zwischen zwei Aminogruppen mindestens zwei Kohlenstoffatome angeordnet sind,

erhalten werden.

Die Bezeichnung (cyclo)aliphatisch soll bedeuten, daß in den Kohlenwasserstoffresten sowohl offenkettig aliphatische als auch (cyclo)aliphatische Struktureinheiten vorliegen können, wobei die Aminogruppen sowohl aliphatisch als auch cycloaliphatisch gebunden sein können. Ein typisches Beispiel eines geeigneten Triamins ist 1,8-Diamino-4-(aminomethyl)octan.

Erfindungsgemäß können weiterhin Ether(poly)isocyanate der Formel (V)

$$X(-R^1\text{-}O\text{-}R^2\text{-}NCO)n \qquad (V),$$

durch Phosgenierung der entsprechenden Ether(poly) amine der Formel (VI)

$$X(-R^1\text{-}O\text{-}R^2\text{-}NH_2)n \qquad (VI),$$

in welchen

X für H, $NH_2$, $C(R^3)4\text{-}n$, $R^1$, $R^2$, $R^3$ für gleiche und verschiedene, gegebenenfalls verzweigte, gegebenenfalls mit Cl-, Br- substituierte, gegebenenfalls Heteroatome (N,O,S) aufweisende $C_1$-$C_{10}$-Alkyl, $C_3$-$C_{24}$-Cycloalkyl, $C_7$-$C_{24}$-Aralkyl, $C_6$-$C_{24}$-Reste steht. $R^1$ kann auch für eine direkte Bindung von X zum an $R^2$-gebundenen Ethersauerstoff stehen, n steht für die Zahl 1,2 und 3,

erhalten werden.

In dem erfindungsgemäßen Verfahren kann ein Mikrostrukturmischer, wie z.B. in der WO 95 304 76, DE-A 195 41 266 oder DE-A 195 40 292 beschrieben oder verwandte, die nach dem gleichen Prinzip arbeiten, eingesetzt werden.

Bei Einsatz eines solchen Mikrostrukturmischers werden die Eduktströme und ein eventueller Inertgasstrom fein zerteilt und feine Fluidfäden erzeugt, die in den Mischbzw. Reaktionsraum als Freistrahlen austreten, ohne daß die Ströme innerhalb des Mikrostrukturmischers miteinander in Kontakt kommen.

Bei der Durchführung des erfindungsgemäßen Verfahrens erfolgt die Mischung mit einem Mikrostrukturmischer, bei dem die Edukte Phosgen und Amin durch eine ihnen jeweils zugeordnete Schar von Mikrokanälen in räumlich getrennte Fluidfäden aufgeteilt werden, und die Fluidfäden der Edukte Amin und Phosgen als Freistrahlen mit für das jeweilige Edukt gleichen Strömungsgeschwindigkeiten in den Misch-/Reaktionsraum austreten, wobei jeder Freistrahl des Edukts Phosgen in unmittelbarer Nachbarschaft zu einem Freistrahl des Edukts Amin in den Misch- und Reaktionsraum geführt wird und sich die benachbarten Freistrahlen durch Diffusion und/oder Turbulenz vermischen.

Das Verfahren kann unter Inertgas (z.B. $N_2$) durchgeführt werden.

Der Mikrostrukturmischer wird so betrieben, daß vorzugsweise laminare Strömungsbedingungen herrschen.

Besonders bewährt hat sich eine Ausführungsform, bei der die Gasfäden der Edukte in abwechselnd übereinanderliegenden oder nebeneinander liegenden Schichten in den Misch-/Reaktionsraum austreten und die Dicke der Fluidfäden der Edukte am Eintritt in den Misch-/Reaktionsraum auf einen Wert zwischen 10 µm und 1000 µm, vorzugsweise 50 µm bis 150 µm eingestellt wird.

Der eingesetzte Mikrostrukturmischer ermöglicht, die Zeit für den turbulenten Wirbelzerfall einzusparen und dadurch den Vermischungsprozeß wesentlich zu beschleunigen.

Besonders bewährt hat sich ein Verfahren, bei dem die Edukte isokinetisch, d. h. mit gleichen Strömungsgeschwindigkeiten die Kanäle des Mikrostrukturmischers verlassen. Dann nämlich werden Rückströmungen vermieden und Belag- sowie Feststoffbildungen, die zu einem Verstopfen des Bauteils führen, können nicht an den Kanalaustrittsöffnungen der Mikrostrukturmischer auftreten.

Eine Alternative zur Vermeidung von Rückströmung und Vermischung der Eduktkomponenten an den Kanalaustrittsöffnungen der Mikrostrukturmischer ist ein Verfahren, bei dem eine Trennschicht aus Inertgas jeweils zwischen den Fluidfäden der Edukte Amin und Phosgen eingesetzt wird, wie in WO 9 530 476 beschrieben ist.

Bei der Phosgenierung polyfunktioneller Amine, z.B. Diaminen, entsteht als Zwischenprodukt eine Verbindung, die neben der entstandenen Isocyanatgruppe noch eine Amingruppe enthält. Diese Verbindung reagiert mit dem Edukt Diamin in einer konkurrenzierenden Folgereaktion zur Phosgenierung zu einem Harnstoff-Derivat.

Erfolgt die Vermischung der Edukte Diamin und Phosgen nur langsam, so liegen während der vergleichsweise langen Mischzeit lokale Überkonzentrationen der Edukte vor und einfach phosgeniertes Diamin

ist stellenweise von hohen Konzentrationen an Diaminen umgeben, was dort zur bevorzugten Bildung von Harnstoff-Derivaten führt. Zur Vermeidung dieses Effekts, der aus der langsamen Vermischung resultiert, wird nach dem Stand der Technik mit hohen Überschüssen an Phosgen gearbeitet.

Eine Beschleunigung des Mischvorgangs verringert die Mischzeit, in der lokale Überkonzentrationen vorliegen, und ermöglicht so eine Reduzierung des Phosgenüberschusses. Der Vorteil dabei ist, daß eine entsprechend kleinere Menge des überschüssigen, hochgiftigen Phosgens abgetrennt und rezykliert werden muß.

Weitere Vorteile sind:

- schnellere Vermischung und damit höhere Ausbeute
- Verminderung des Anteils an Nebenprodukten und damit weniger Feststoffbildung, die zu Verstopfungen im Reaktionsraum und dem Mischer führen kann.
- dadurch Erhöhung der Standzeit

**Beispiele**

Die Figuren beschreiben im Einzelnen:

Fig. 1 den prinzipiellen Aufbau einer bevorzugten Ausführungsform des Mikro-strukturmischers für zwei Edukte A, B mit symmetrischen Strömungswegen,

Fig. 2 die Vermischung der aus dem Mikrostrukturmischer in den Misch- bzw. Reaktionsraum eintretenden, den Edukten A, B zugeordneten Freistrahlen,

Fig. 3 ein Fließschema für eine Apparatur zur Untersuchung von Gasphasenphosgenierungen unter Einsatz eines Mikrostrukturmischers.

In Fig. 1 ist eine typische Ausführung eines Mikrostruktur-Mischers gemäß WO 9 530 476 schematisch dargestellt. Das Bauprinzip dieses Mischers beruht darauf, daß verschiedene Lagen der Platten mit schrägverlaufenden Mikrokanälen vertikal übereinander gestapelt sind.

Auf eine Platte mit den Mikrokanälen 1a folgt jeweils eine Platte mit den Mikrokanälen 1b, d.h. zwei im Stapel unmittelbar übereinander angeordnete Platten sind jeweils mit einer Schar von Mikrokanälen 1a, 1b versehen, wobei die Mikrokanalscharen aufeinanderfolgender Platten einen Winkel $\alpha$ miteinander bilden und symmetrisch zur Horizontalachse in Fig. 1, d.h. spiegelbildlich zueinander angeordnet sind. Die Platten haben z.B. eine Dicke von 100 $\mu$m. Die Querschnittsabmessungen der Mikrokanäle liegen typischerweise in der Größenordnung 70 bis 200 $\mu$m.

Die in Fig. 1 von der Bildmitte aus gesehen schräg nach oben verlaufenden Scharen von Mikrokanälen 1a münden linksseitig in eine Verteilerkammer 3a, der ein Edukt A zugeführt werden kann. Analog münden die schräg nach unten verlaufenden Scharen der Mikrokanäle 1b linksseitig in eine Verteilerkammer 3b, der ein Edukt B zugeführt werden kann. Beide Scharen von Mikrokanälen münden rechtsseitig, ohne sich zu durchkreuzen, in einen gemeinsamen Misch-/Reaktionsraum 4 ein. Die spiegelsymmetrische Anordnung der Mikrokanäle 1a, 1b ist nicht zwingend erforderlich. Die Mikrokanäle 1b können z.B. auch eine andere Neigung gegen die Horizontalachse haben als die Mikrokanäle 1a.

Wichtig ist jedoch, daß die Mikrokanäle einer Schar jeweils strömungstechnisch untereinander gleich sind, d.h. daß die Mikrokanäle 1a alle den gleichen Strömungswiderstand besitzen. Die gleiche Bedingung gilt für den Strömungswiderstand der Mikrokanäle 1b, wobei aber die Strömungswiderstände der beiden Mikrokanalscharen 1a, 1b (im Verhältnis zueinander) unterschiedlich sein können. Gleicher Strömungswiderstand kann dadurch erreicht werden, daß die Länge und der Querschnitt für alle Mikrokanäle 1a gleich sind.

Das einer Verteilerkammer 3a, 3b zugeführte Edukt, z.B. ein gasförmiger Reaktand, verteilt sich jeweils auf die Mikrokanäle 1a, 1b. Die Zusammenführung der beiden Reaktanden erfolgt beim Eintritt in den Misch-/Reaktionsraum und wird im folgenden anhand der Fig. 2 näher beschrieben.

In Fig. 2 ist der Mündungsquerschnitt des Mikrostrukturmischers perspektivisch dargestellt.

In der obersten Lage oder Platte münden z.B. die dem Edukt A zugeordneten Mikrokanäle 1a und in der darauffolgenden darunterliegenden Lage oder Platte die Mikrokanäle 1b des Eduktes B in den Misch-/Reaktionsraum ein. Darauf folgt wieder eine Lage oder Platte mit den zum Edukt A gehörenden Mikrokanälen usw.. In Fig. 2 ist auch schematisch dargestellt, wie die in den Mikrokanälen geführten Fluidströme als Freistrahlen 6a, 6b in den Misch-/Reaktionsraum eintreten und sich mit zunehmender Entfernung von der Mündung untereinander vermischen. Die Mischung erfolgt dabei durch Diffusion und/oder Turbulenz, während in den Mikrokanälen in der Regel laminare Strömungsbedingungen vorherrschen. Gleichzeitig mit der Mischung setzt auch die Reaktion der Edukte A, B ein. Das Reaktionsprodukt wird am Ende der Misch-/Reaktionskammer abgenommen (s. Fig. 1).

Der Mikrostrukturmischer gemäß Fig. 1 kann auch in der Weise modifiziert werden, daß neben den Edukten Phosgen und Amin ein Inertgas durch den Mikromischer in Form von Fluidfäden zu dem Misch-/Reaktionsraum geführt wird. Dabei werden die Fluidfäden des Inertgases so geführt, daß in Nachbarschaft zu einem Freistrahl eines Edukts stets ein Freistrahl des Inertgases in den Misch-/Reaktionsraum eingespeist wird.

**Ausführungsbeispiel**

Als Beispiel für eine Gasphasenphosgenierung wurde die Phosgenierung von H6-TDA (1-Methyl-2,4-diamino-cyclohexan) zu H6-TDI (1-Methyl-2,4-diisocyanato-cyclohexan) untersucht.

Dabei läuft die folgende Hauptreaktion in der homogenen Gasphase bei 350°C ab:

$$\text{H6-TDA} + 2\ \text{Phosgen} \rightarrow \text{H6-TDI} + 4\ \text{Chlorwasserstoff}$$

Die Untersuchungen wurden in der in Fig. 4 dargestellten Versuchsapparatur durchgeführt.

Sie besteht aus den Dosierventilen 1 für Phosgen und Amin, den Überhitzern 2, in denen die Edukte auf Reaktionstemperatur gebracht werden, den Feinfiltern 3, dem Mikrostrukturmischer 4, und einem Verweilzeitrohr 5, in dem die Reaktion stattfindet. Der eingesetzte Mikrostrukturmischer verfügt über Kanäle der Dicke 70 μm, einer Kanalbreite von 100 um und einer Kanallänge von 14 mm. Dabei ist der Mischer aus 50 Folien mit jeweils 50 Kanälen pro Strömungspassage hergestellt. Der Vergleich erfolgte mit einer konventionellen Glattstrahldüse im Labor.

Um eine isokinetische Fahrweise des Mikrostrukturmischers und gleichzeitig einen Überschuß an Phosgen von 200% zu realisieren, wurden folgende Molenströme eingestellt:

- Phosgen: 6 mol/h
- Diamin + Stickstoff: 1 mol/h + 5mol/h = mol/h

Bei den Versuchen mit der konventionellen Glattstrahldüse im Labor wurde eine Ausbeute von 97 % an 1-Methyl-2,4-diisocyanato-cyclohexan erzielt. Bei Einsatz des Mikrostrukturmischers ergab sich dagegen eine Ausbeute von 99%. Die in der EP-A 676 392 angegebene Ausbeute beläuft sich auf 96 %.

Damit zeigt sich, daß bei Einsatz eines Mikrostrukturmischers für die Gasphasenphosgenierung wesentlich weniger Nebenprodukte wegen der schnelleren Vermischung gebildet werden.

**Patentansprüche**

1. Verfahren zur Herstellung von Isocyanaten durch Phosgenierung der entsprechenden Amine in der Gasphase unter Einsatz eines Mikrostrukturmischers zur schnellen Vermischung der Amine mit Phosgen, **dadurch gekennzeichnet, daß** die Mischung mit einem Mikrostrukturmischer erfolgt, bei dem die Edukte Phosgen und Amin durch eine ihnen jeweils zugeordnete Schar von Mikrokanälen in räumlich getrennte Fluidfäden aufgeteilt werden, die anschließend in einen gemeinsamen Misch- und Reaktionsraum austreten, wobei die Fluidfäden der Edukte Amin und Phosgen als Freistrahlen mit für das jeweilige Edukt gleichen Strömungsgeschwindigkeiten in den Misch-/Reaktionsraum austreten, wobei jeder Freistrahl des Edukts Phosgen in unmittelbarer Nachbarschaft zu einem Freistrahl des Edukts Amin in den Misch- und Reaktionsraum geführt wird und sich die benachbarten Freistrahlen durch Diffusion und/oder Turbulenz vermischen.

2. Verfahren nach Anspruch 1, **dadurch gekennzeichnet, daß** als Isocyanate, Diisocyanate, Triisocyanate und/oder Ether(poly)isocyanate erhalten werden.

3. Verfahren nach Anspruch 1, **dadurch gekennzeichnet, daß** als Amine Diamine, Triamine und/oder Ether(poly)amine eingesetzt werden.

4. Verfahren nach Anspruch 1, **dadurch gekennzeichnet, daß** die Eduktströme mit einem Inertgas verdünnt sind.

5. Verfahren nach Anspruch 1, **dadurch gekennzeichnet, daß** in den Strömungskanälen im Mikrostrukturmischer laminare Strömungsbedingungen für die Edukte aufrecht erhalten werden.

6. Verfahren nach Anspruch 1, **dadurch gekennzeichnet, daß** die Fluidfäden der Edukte in abwechselnd übereinanderliegenden oder nebeneinander liegenden Schichten in den Misch-/Reaktionsraum austreten.

7. Verfahren nach Anspruch 1, **dadurch gekennzeichnet, daß** die Dicke der Fluidfäden der Edukte am Eintritt in den Misch-/Reaktionsraum auf einen Wert zwischen 10 μm und 1000 μm, vorzugsweise 50 μm bis 150 μm eingestellt wird.

8. Verfahren nach Anspruch 1 und 6, **dadurch gekennzeichnet, daß** in die Nachbarschaft zum Fluidfaden eines Edukts zusätzlich ein Fluidfaden eines Inertgases geführt wird.

9. Verfahren nach Anspruch 6, **dadurch gekennzeichnet, daß** die Edukte isokinetisch aus dem Mikrostrukturmischer austreten.

**Claims**

1. Process for the preparation of isocyanates by phosgenation of the corresponding amines in the gas phase using a microstructure mixer for rapid mixing of the amines with phosgene, **characterized in that** the mixing is carried out with a microstructure mixer in which the educts phosgene and amine are divided by a system, assigned to each of them, of microchannels into spatially separated fluid threads which then emerge into a common mixing and reaction space, the fluid threads of the educts amine and phosgene emerging as free jets with the same flow rates for the particular educt into the mixing/reaction space, each free jet of the educt phosgene being led into the mixing and reaction space immediately adjacent to a free jet of the educt amine and the adjacent free jets mixing with one another by diffusion and/or turbulence.

2. Process according to claim 1, **characterized in that** diisocyanates, triisocyanates and/or ether(poly)isocyanates are obtained as the isocyanates.

3. Process according to claim 1, **characterized in that** diamines, triamines and/or ether-(poly)amines are employed as the amines.

4. Process according to claim 1, **characterized in that** the educt streams are diluted with an inert gas.

5. Process according to claim 1, **characterized in that** laminar flow conditions for the educts are maintained in the flow channels in the microstructure mixer.

6. Process according to claim 1, **characterized in that** the fluid threads of the educts emerge into the mixing/reaction space in layers lying alternately one above the other or side by side.

7. Process according to claim 1, **characterized in that** the thickness of the fluid threads of the educts on entry into the mixing/reaction space is adjusted to a value between 10 μm and 1,000 μm, preferably 50 μm to 150 μm.

8. Process according to claim 1 and 6, **characterized in that** a fluid thread of an inert gas is additionally fed adjacent to the fluid thread of an educt.

9. Process according to claim 6, **characterized in that** the educts emerge isokinectically from the microstructure mixer.

**Revendications**

1. Procédé pour la préparation d'isocyanates par phosgénation des amines correspondantes en phase gazeuse avec utilisation d'un mélangeur à microstructure pour un mélange rapide des amines avec le phosgène, **caractérisé en ce que** le mélange est réalisé à l'aide d'un mélangeur à microstructure dans lequel les composants de départ, phosgène et amine, sont répartis par un système de microcanaux particulier pour chacun d'eux en filaments de fluide séparés dans l'espace, qui sortent ensuite dans une chambre commune de mélange et de réaction, les filaments de fluide des composants de départ amine et phosgène entrant dans la chambre de mélange/réaction sous la forme de jets libres s'écoulant à une vitesse égale pour chacun des composants de départ, chaque jet libre du composant phosgène entrant dans la chambre de mélange et de réaction au voisinage immédiat d'un jet libre du composant amine, et les jets libres voisins se mélangeant alors par diffusion et/ou turbulence.

2. Procédé selon la revendication 1, **caractérisé en ce que** les isocyanates obtenus sont des diisocyanates, des triisocyanates et/ou des éther(poly)isocyanates.

3. Procédé selon la revendication 1, **caractérisé en ce que** les amines mises en oeuvre sont des diamines, des triamines et/ou des éther(poly)amines.

4. Procédé selon la revendication 1, **caractérisé en ce que** les courants de composants de départ sont dilués par un gaz inerte.

5. Procédé selon la revendication 1, **caractérisé en ce que** dans les canaux d'écoulement du mélangeur à microstructure, on maintient pour les composants de départ des conditions d'écoulement laminaire.

6. Procédé selon la revendication 1, **caractérisé en ce que** les filaments de fluides des composants de départ pénètrent dans la chambre de mélange/réaction sous forme de couches superposées ou contiguës alternantes.

7. Procédé selon la revendication 1, **caractérisé en ce que** l'épaisseur des filaments de fluide des composants de départ à l'entrée dans la chambre de mélange/réaction est réglée à un niveau de 10 à 1000 μm, de préférence de 50 à 150 μm.

8. Procédé selon les revendications 1 à 6, **caractérisé en ce que**, au voisinage des filaments de fluides d'un composant de départ, on amène en outre un filament de fluide d'un gaz inerte.

9. Procédé selon la revendication 6, **caractérisé en ce que** les composants de départ sortent du mélangeur à microstructure sous forme isocinétique.

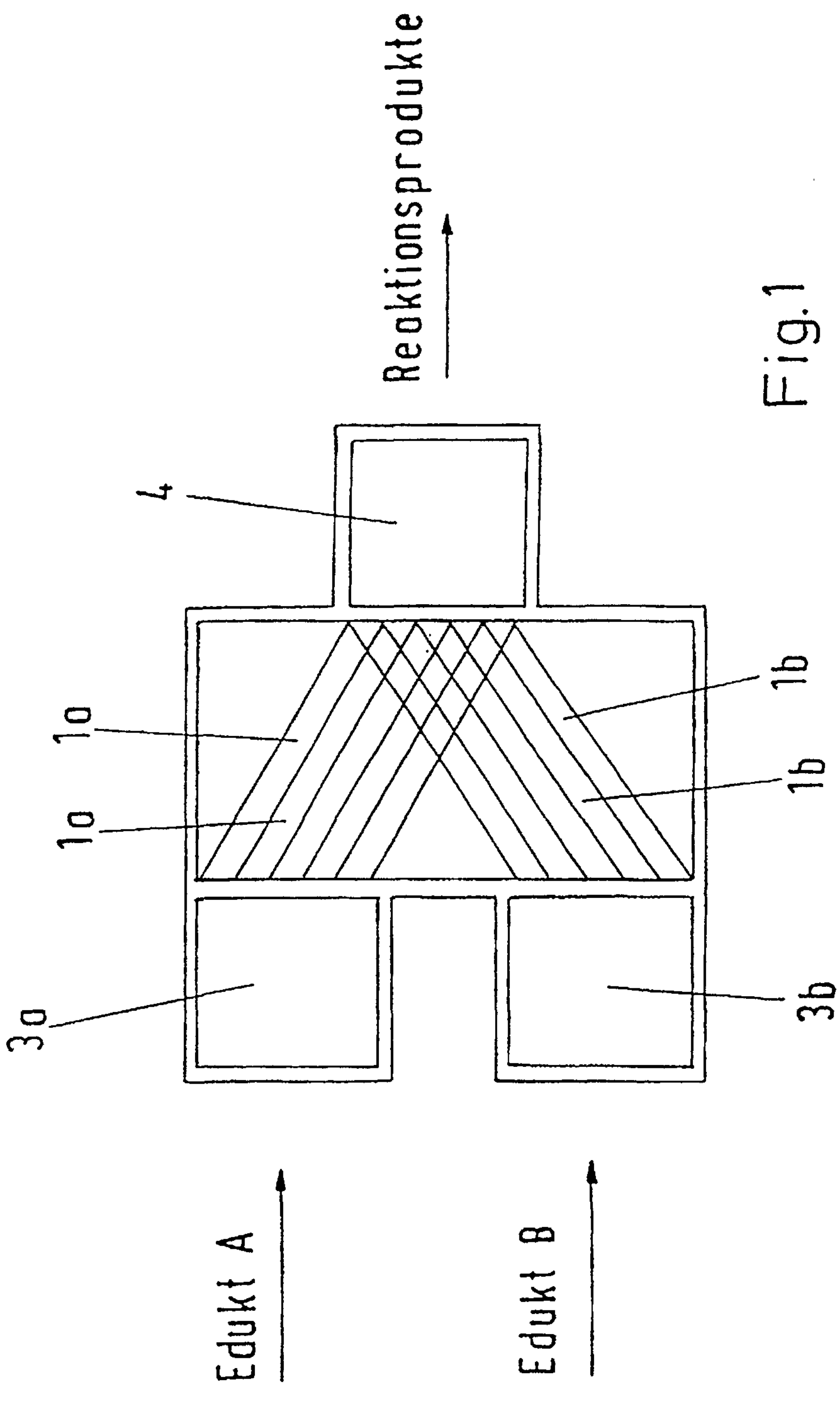


Fig.1

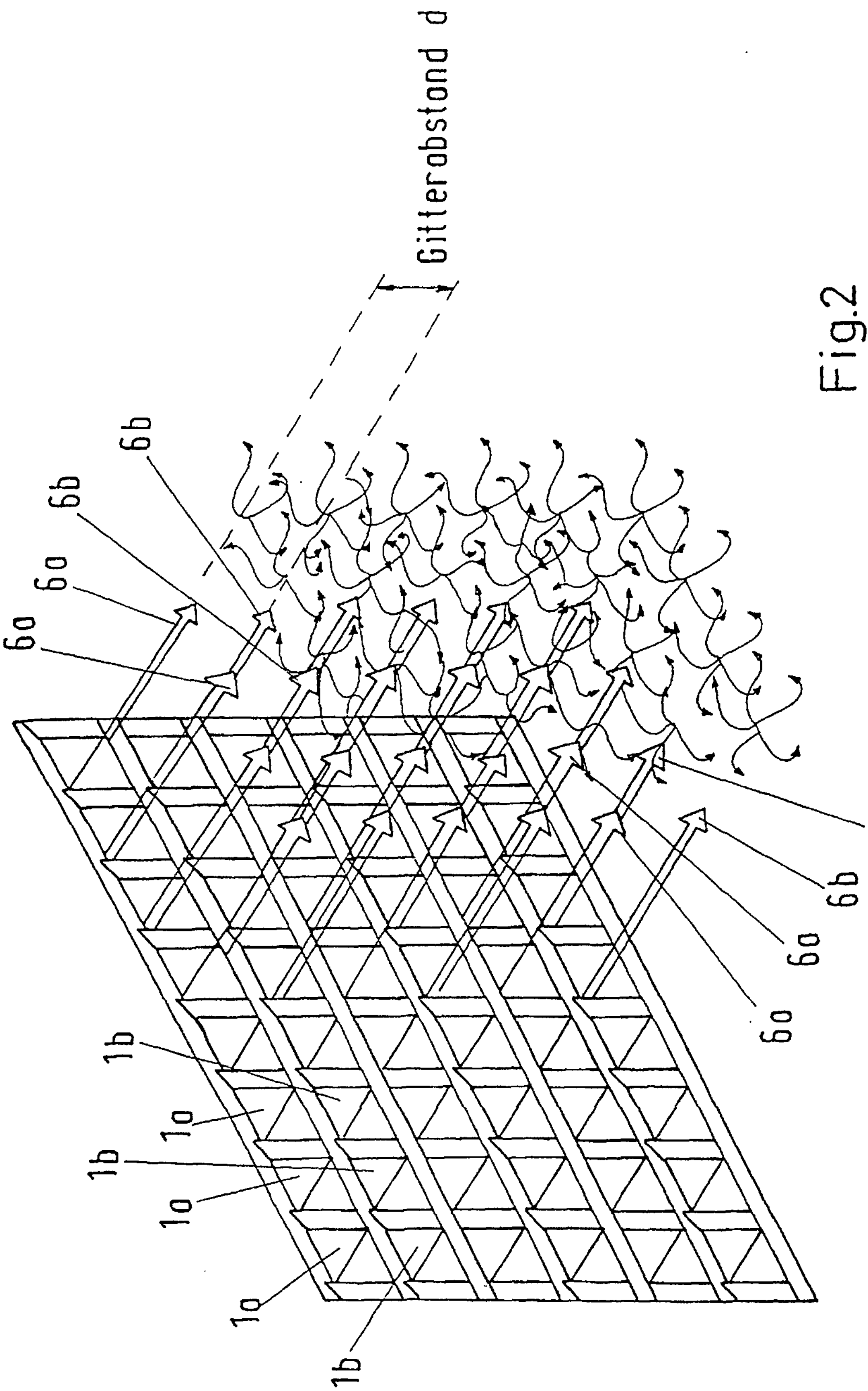


Fig.2

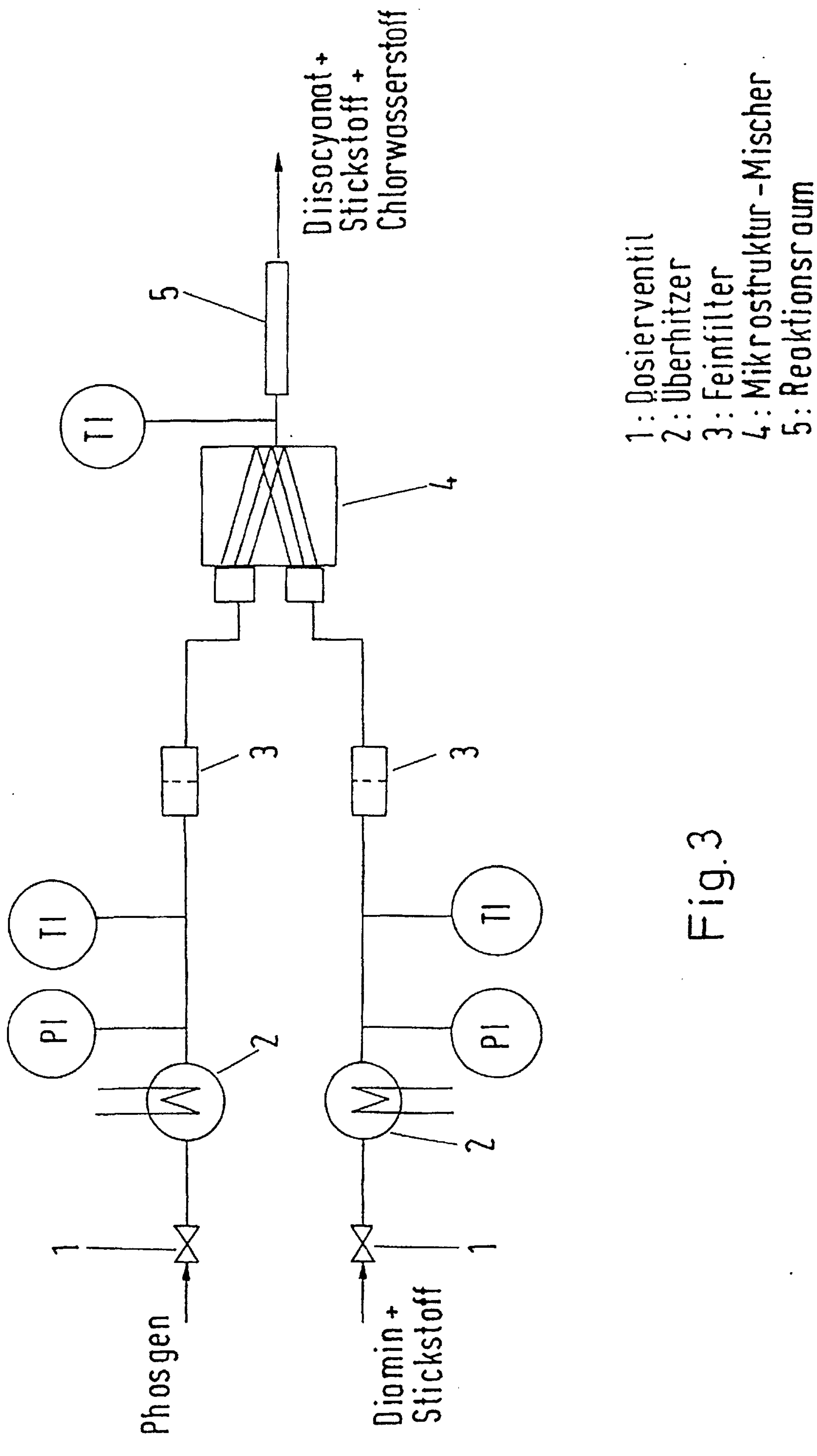
Diisocyanat +
Stickstoff +
Chlorwasserstoff
Phosgen
Diamin +
Stickstoff
TI
PI
1
2
3
4
5
1: Dosierventil
2: Überhitzer
3: Feinfilter
4: Mikrostruktur-Mischer
5: Reaktionsraum

Fig. 3